# EUROPEAN PATENT SPECIFICATION

(11) **EP 2 711 642 B1**
(45) Date of publication and mention of the grant of the patent: **14.01.2015**
(21) Application number: 12185990.4
(22) Date of filing: 25.09.2012
(51) Int. Cl.: F24F 3/16, B01D 53/79, A61L 9/14, F24F 3/044

(54) **Air supply of a manufacturing site**
Luftversorgung einer Herstellungsanlage
Alimentation en air d'un site de fabrication

(43) Date of publication of application: 26.03.2014
(73) Proprietor: Airbus Operations GmbH, 21129 Hamburg (DE)
(72) Inventor: Hottendorf, Gerd, 21756 Osten (DE); Engelmann, Axel, 21698 Harsefeld (DE); Wilms, Matthias, 27478 Cuxhaven (DE); Hellwege, Heinz, 21745 Hemmoor (DE)
(74) Representative: Maiwald Patentanwalts GmbH

(56) References cited:
- WO-A1-2010/073217
- US-A- 5 090 972
- US-A- 5 707 005
- US-A1- 2011 064 607
- US-A1- 2011 265 648

## Description

### FIELD OF THE INVENTION

The present invention relates to supplying air to a manufacturing site, and relates in particular to an air supply system for a manufacturing site, and a method for air supply of a manufacturing site.

### BACKGROUND OF THE INVENTION

In manufacturing sites, for example for the manufacture of aircrafts or vehicles, the supply of air with predetermined parameters may be requested, such as the supply of air within a certain temperature range, and air having a relative humidity within a predefined range. Further, air supply may also be used to remove used air, or even to remove contaminated air, that has been contaminated during certain manufacturing steps, for example during painting processes or certain machining processes, such as grinding steps. Due to the energy awareness becoming increasingly important over the last years, exhaust air may be used for the recovery of thermal energy to improve the energy efficiency. However, it has been shown that contaminated air, e.g. contaminated with volatile organic compounds (VOC) may have a negative effect on the efficiency of an air supply for a manufacturing site, i.e. on the energy efficiency of the system, since the use of the exhaust air is limited due to the contamination.

WO 2010/073217 Ail relates to an air-purifying system and a respective method. A system is provided for purifying air in an indoor environment with subsequent total or partial recirculation or ejection outside. Aspirating means are provided that are adapted to generate a flow of air circulating along a path defined by convey means. Further, purifying means are arranged along the path. The purifying means comprise at least one scrubbing unit of the air by passing the flow through a liquid with controlled *pH* and added ozone. Further, a unit for generating and feeding the ozone to the scrubbing unit is provided.

### SUMMARY OF THE INVENTION

Thus, there is a need to provide an air supply for a manufacturing site with improved energy efficiency, where contaminated air can also be handled.

The object of the present invention is solved by the subject-matter of the independent claims, wherein further embodiments are incorporated in the dependent claims. It should be noted that the following described aspects of the invention apply also for the air supply system for a manufacturing site, as well as for the method for air supply of a manufacturing site.

According to a first aspect, an air supply system for a manufacturing site is provided that comprises a desiccant and evaporative cooling arrangement, an exhaust air scrubber, and first supply air conduits from the desiccant and evaporative cooling arrangement to at least one contaminated air generating indoor space, as well as secondary disposal conduits from the at least one contaminated air generating indoor space to the exhaust air scrubber and to the desiccant and evaporative cooling arrangement. The desiccant and evaporative cooling arrangement is configured to condition fresh outdoor air as input air for the use in the contaminated air generating indoor space. The first air supply conduits are configured to provide the conditioned air as supply air to the at least one contaminated air generating indoor space, and the secondary disposal conduits are configured to conduct contaminated exhaust air from the at least one contaminated air generating indoor space to the exhaust air scrubber. The secondary disposal conduits are also configured to provide the scrubbed air to the desiccant and evaporative cooling arrangement. The exhaust air scrubber is configured to remove air contamination to a predefined amount, wherein the predefined amount is set such that the air can be used further in the desiccant and evaporative cooling arrangement. The scrubbed air is used for at least one of the group of: cooling the input air by heat recovery, and desiccating a drying agent of the desiccant and evaporative cooling arrangement.

As an advantage, a combined air conditioning and venting system is provided, where the contamination in the exhaust air is removed. For example, the exhaust air can be generated in a paint shop. Providing the exhaust air scrubber makes it possible to use a desiccant evaporative cooling, which is a highly efficient concept for providing cooling thermal energy, for example for use in air conditioning systems. Providing the exhaust air scrubber also results in that the exhaust air that is leaving the system has very low emissions.

For example, the air contamination is removed by at least a factor of 80 %, or even 95 %. For example, the air leaving de-contamination is suitable to be provided for further purposes, such as transfer of thermal energy in a heat recovery arrangement or the like.

According to the invention, different zones with different air conditioning requirements are provided, wherein, in addition to the at least one contaminated air generating indoor space, at least one air-conditioned indoor space is provided. The first air supply conduits are provided from the desiccant and evaporative cooling arrangement to the at least one air-conditioned indoor space. Secondary air supply conduits are provided from the at least one air-conditioned indoor space to an air supply arrangement, and from the air supply arrangement to the at least one contaminated air generating indoor space. The desiccant and evaporative cooling arrangement is configured to condition fresh outdoor air as input air for the use in the air-conditioned indoor spaces. The air supply arrangement is configured to condition exhaust from the at least one air-conditioned indoor space for the use in the contaminated air generating space. The first air supply conduits are configured to provide the conditioned air as supply air to the at least one air-conditioned indoor space, and the secondary air disposal conduits are configured to conduct exhaust air from the at least one air-conditioned indoor space to the air supply arrangement, and to provide the conditioned air as supply air to the at least one contaminated air generating indoor space. The different zones may relate to a first and second type of room. The latter referring to rooms in which air is generated that shows contamination making it unsuitable for further use; the first type relating to rooms in which air is used such that the exhaust air can be used for purposes such as re-use of thermal-energy

As an advantage, the prepared and preconditioned supply air is first supplied to the air-conditioned indoor spaces, in which the air is not contaminated, or at least not essentially contaminated. The exhaust air from the air-conditioned indoor space is then supplied to those spaces, in which contaminated air is generated, so-to-speak as a second use. For example, the air-conditioned indoor spaces may require cooler air, than the contaminated air generating indoor spaces.

This is in contrast to conventional cooling, where air is cooled in a first step, whereby the relative humidity reaches nearly the absorption point of 100 % relative humidity; the air is then heated up in a second step to a certain amount to achieve the desired temperature and an acceptable relative humidity.

The air-conditioned indoor space may also be referred to as primary air-conditioned indoor space, and the contaminated air generating space may be referred to as contaminated air generating secondary indoor space. The desiccant and evaporative cooling arrangement may then be referred to as primary desiccant and evaporative cooling arrangement, and the air supply arrangement as secondary air supply arrangement.

The desiccant and evaporative cooling arrangement may also be referred to as DEC arrangement. Further, also the term DCS (desiccant cooling system) may be used. The basic principle is cooling by drying and evaporation, i.e. evaporative cooling. Warm input air is dehumidified or dried in, for example, a rotational dryer or sorption-wheel. Thereby, the enthalpy is increasing, and thus the temperature, and the humidity ratio (or water concentration) is decreasing. The air is then cooled, for example in a cross-stream heat exchanger. The input air (to be cooled) transfers thermal energy to the exhaust air of the system. Further, the air is humidified, by which the air temperature decreases. The air can then be supplied. For a regeneration of the rotational dryer or sorption-wheel, exhaust air is heated such that the exhaust air can absorb humidity from the drying agent provided in the rotational dryer/sorption-wheel.

The DEC arrangement may comprise a plurality of DEC units, which may be connected via a common supply duct of the air conditioned space to a plurality of supply openings of the contaminated air generating space. Further, a common exhaust discharge duct is provided to collect the contaminated air and to and supply the exhaust air. The air supply arrangement may comprise a plurality of units connected to a common secondary air supply conduit. The plurality of units may also be provided with connections to a respective space unit. The cooling with a DEC system comprises the basic steps: i) drying the air in the rotational dryer or sorption-wheel, which leads to dryer, but warmer air; ii) heat recovery for cooling the air, by basically maintaining the humidity, i.e. increasing the relative humidity; iii) humidifying and cooling the air by a rather small degree to arrive at optimized air condition parameters.

According to an exemplary embodiment, it is provided at least one air-conditioned indoor space, and/or at least one contaminated air generating indoor space.

For example, the air-conditioned indoor spaces comprise manufacturing halls, and the contaminated air generating indoor spaces comprise painting cabins or painting rooms.

According to an exemplary embodiment, the desiccant and evaporative cooling arrangement comprising a drying agent provided in a sorption-generator. At least a part of the heat for the cyclic recovery of the drying agent is provided by low thermal energy from a combined heat and power plant.

For example, a part of the heat for the cyclic recovery of the drying agent is provided by one or more heat pumps. In another example, solar energy provides thermal energy for the cyclic recovery.

According to an exemplary embodiment, the desiccant and evaporative cooling arrangement comprises at least one dry wheel with a drying agent, which dry wheel is arranged within the first air supply duct with a first portion and within the secondary disposal conduit with a second portion. Further, at least one heat recovery wheel is provided, which is arranged within the first air supply duct with a first portion and within the secondary disposal conduit with a second portion.

According to an exemplary embodiment, at least one air heater is provided between the heat recovery wheel and the dry wheel in the secondary disposal conduit.

According to an exemplary embodiment, the exhaust air scrubber is an integrated unit of the desiccant and evaporative cooling arrangement.

According to a second aspect, a method for air supply of a manufacturing site is provided, comprising the following steps:
a) conditioning of fresh outdoor air in a desiccant and evaporative cooling arrangement as input air for the use in contaminated air generating indoor spaces; and providing the conditioned air as supply air to at least one contaminated air generating indoor space;
b) conducting of contaminated exhaust air from the at least one contaminated air generating indoor space to an exhaust air scrubber where water is sprayed to i) humidify and cool the air, and to ii) remove air contamination to a predefined amount, wherein the predefined amount is set such that the air can be used further in the desiccant and evaporative cooling arrangement; and
c) providing the scrubbed air to the desiccant and evaporative cooling arrangement, wherein the scrubbed air is used for at least one of the group of: cooling the input air by heat recovery, and desiccating a drying agent of the desiccant and evaporative cooling arrangement.

According to the invention, the manufacturing site comprises different zones with different air conditioning requirements, wherein in step a) the fresh outdoor air is conditioned for the use in air-conditioned indoor spaces, and the conditioned air is provided as supply air to at least one air-conditioned indoor space. Between step a) and step b), the following steps are provided:
ab1) conducting of exhaust air from the at least one air-conditioned indoor space to an air supply arrangement of contaminated air generating spaces; and
ab2) conditioning of the exhaust air in the air supply arrangement for the use in contaminated air generating spaces, and providing the conditioned air as the supply air to the at least one contaminated air generating indoor space.

In step b), contaminated exhaust air from the at least one contaminated air generating indoor space is conducted to the exhaust air scrubber.

In one example, step ab1) is referred to as step b), and step ab2) as step c), and step b) as step d), and step c) as step e).

The water used for the spraying in the scrubber may be non-treated drinking water to which a pH-stabilizer (*Härtestabilisator*) has been added, and which has been sterilized with a UV light arrangement, e.g. a UV tube lamp. In another example, clean and treated water is provided.

The different zones may comprise a first type of zone and a second type of zone. The first type may relate to rooms or other enclosed spaces where air supply has to be provided within predetermined values, i.e. with requirements for air conditioning, e.g. air with a temperature and relative humidity predefined, and may be also air purity, within a respective predetermined range and where no substantial contamination occurs that might restrict the further use of the respective exhaust air. The second type may relate to rooms or other enclosed spaces, where air is polluted or contaminated and needs to be replaced or refreshed in a predetermined range of air exchange rate, and which air is contaminated such that a direct further use in an air supply system is not possible without further measurements. In another example, office spaces, or laboratory spaces, are provided as air-conditioned indoor spaces. The first type comprises manufacturing halls, office spaces and the like. The manufacturing sites may be provided for mounting and assembly of vehicles, aircrafts, coaches, trains, or production of parts thereof. The second type may also have certain requirements relating to air temperature and relative humidity, and may be also air purity. The second type comprises paint-shops, machining and production arrangements and the like. The contaminated air generating indoor spaces comprise painting cabins or paint shops, cleaning cabins, or mounting spaces using solvent substances, such as organic solvents. The contaminated air generating spaces may also comprise spaces or cabins where aerosols are used and/or generated in production or treatment processes. The contaminated air generating spaces may also comprise spaces or cabins where small particles, such as nano-particles, are used and/or generated in respective processes, and which particles then contaminate the air, which thus requires to be replaced rather frequently. The particles may also comprise dust particles.

The input air is blown into the at least one air-conditioned indoor space, for example, via large-distance throwing air nozzles provided in, for example, approximately 4 m height. In step d), the exhaust air may be discharged via air suction openings arranged in the floor area, for example by floor grids or floor gratings.

For example, in step a), the air is first dried and thereby heated up to approximately 45 °C. By heat exchange, the air is then cooled to approximately 10 to 22 °C. A further cooling is provided by, for example, a cooling register, leading to an air temperature of approximately 18 °C, which is blown into the air-conditioned indoor space. In the latter, the air may be heated up to approximately 21 °C.

If the air-conditioned indoor space is an assembly hall, also other values can be predefined, according to the assembly steps performed inside the assembly hall. For example in step c), the air is conditioned to a range suitable for painting processes, e.g. a temperature range of approximately 20 to 25 °C, and a relative humidity range of approximately 40 to 70 %.

The amount of preconditioning by the air supply arrangement may depend on a state of the exhaust air from the air condition indoor space.

According to an exemplary embodiment, the exhaust air from the air-conditioned indoor spaces is used as air source for contaminated air generating indoor spaces. The exhaust air from the contaminated air generating indoor spaces and/or from the air-conditioned indoor spaces is used for operating the desiccant and evaporative cooling arrangement for conditioning the supply air for the contaminated air generating indoor space and/or for the air-conditioned indoor spaces.

According to an exemplary embodiment, the desiccant and evaporative cooling arrangement comprises a drying agent provided in a sorption-generator. The cyclic recovery of the drying agent is supported by low thermal energy from a combined heat and power plant.

The drying agent may be provided in a sorption-wheel or sorption-rotor as sorption-generator.

Following step c), the scrubbed air may be discharged as exit air from the DEC arrangement.

According to an exemplary embodiment, the air contamination is provided by:
i) particles, wherein the amount of sprayed water that is not solved in the air is at least partly collected and supplied to a filter arrangement, and may then be reintroduced to the above described air treatment steps; and/or
ii) volatile organic compounds, wherein the amount of sprayed water that is not solved in the air is at least partly collected and supplied to a biological reactor, where bacteria provide degrading of the volatile organic compounds into CO₂, and may then be reintroduced to the above described air treatment steps.

For example, the volatile organic compounds, also known as VOC, are removed by a degree of at least approximately 70 %, for example 90 % or even 98 %. The cleaned waste water can be provided, for example, to the DEC arrangement for cooling and/or scrubbing purposes.

According to an exemplary embodiment, step a) comprises drying of the input air and simultaneous heating of the input air, cooling of the heated input air by a heat recovery wheel and humidifying of the input air, optionally additional heating. Step ab2) comprises optionally humidifying, and optionally additional heating. Further, step b) comprises humidifying and simultaneous scrubbing by high speed spraying of water onto a surface where the water drops are atomized into smaller droplets generating negative charges, and absorbing the thermal energy from the heat recovery wheel by the air cooled by the humidification, and thereby heating up the air, and optionally heating of the air by low temperature moisture expeller, optionally heating of the air by high temperature moisture expeller for disinfection, and drying of the drying agent by hot air. The generation of negative charges is also known as water fall effect or Lenard effect.

For example, the following steps are provided for a warm summer day, with an air outdoor temperature of approximately 35 °C and 13 gram water per kilogram of dry air, i.e. 30 g/kg. The input air enters the desiccant and evaporative cooling arrangement with 35 °C and 13 g water, i.e. 40 % relative humidity (state 1). By the drying step, for example in an absorption wheel, the air is heated up to approximately 55 °C (state 2). In the following step, the air is cooled to 23 °C (state 3), for example by a heat recovery wheel. By humidifying, the air is further cooled down to 19 °C. Then, a ventilator provides heating of less than 1 °C (state 4). In the air-conditioned indoor space, the air is heated and humidified to 24 °C and 8 g/kg (state 5). After suction of this exhaust air, the air supply arrangement provides humidifying, which cools the air to 20 °C and a humidity of 9.7 g/kg (state 6). The air is supplied to the contaminated air generating indoor space and leaves as (contaminated) exhaust air heated to 25 °C and humidified to 10.5 g/kg (sate 7). The exhaust air is then supplied to exhaust cleaning and heat recovery in the desiccant and evaporative cooling arrangement. Leaving an exhaust humidifier, i.e. the scrubber, the air has as temperature of 19 °C and 13 g/kg (state 8). Simultaneously, negative ions, which are generated in this state, are used for cleaning the exhaust air. Following the humidifier/scrubber, the air provides the cold thermal energy to the heat recovery wheel, i.e. the cold air absorbs thermal energy in the heat recovery wheel, by which is air is heated to 40 °C and 13 g/kg (state 9). A low temperature expeller further heats the air to 55 °C and 13 g/kg (state 10). If necessary, for example for disinfection, by a high temperature expeller the air can be heated up to 100 °C. The hot air with very low relative humidity (approximately 12 %) is then used for drying the absorption agent in the absorption wheel and leaves the system in state 11.

According to an exemplary embodiment, step a) comprises first humidifying and pre-heating of the input air with an absorption wheel, heating with a high temperature heater, and optionally heating with a cooler that also works as a low temperature heater, in relation with the scrubber. Step ab2) comprises increasing of the air temperature by recirculation cooler/heater heating, and second humidifying.

For example, the following steps are provided for a cold winter day, with an air outdoor temperature of approximately -12 °C and 1 gram water per kilogram of dry air. The input air enters the desiccant and evaporative cooling arrangement with -12 °C and 1 g/kg, i.e. approximately 40 % relative humidity (state 1). The air is then humidified and pre-heated by the absorption wheel to approximately 13 °C and 8 g/kg (state 2). The high temperature heater then heats the air to 20 °C (state 3). The cooler can optionally work as a low temperature heater and can heat the air. In the air-conditioned indoor space, the air is heated and humidified to 23 °C and 7 g/kg (state 4), wherein the increased temperature is provided by the circulating air cooler/heater. After suction of this exhaust air, the air supply arrangement provides humidifying to 8 g/kg (state 5). The air is supplied to a contaminated air generating indoor space, and leaves as (contaminated) exhaust air heated to 24 °C and 9 g/kg (state 6). The exhaust air is then supplied to exhaust cleaning and heat recovery in the desiccant and evaporative cooling arrangement. Leaving the exhaust humidifier/scrubber, the air has a temperature of 17 °C and 12 g/kg (state 7). Simultaneously, negative ions that are generated in this stage are used for cleaning the exhaust air. Following the humidifier, the air leaves the system with 2 °C and 4 g/kg (state 8).

According to an exemplary embodiment, step a) comprises first humidifying and pre-heating of the input air with an absorption wheel, heating with a high temperature heater, optionally heating with a cooler that also works as a low temperature heater, in relation with scrubber, humidifying and cooling with input humidifier, and heating with cooler that also works as low temperature heater. Step ab2) comprises increasing of the air temperature by recirculation cooler/heater heating, and second humidifying.

According to an aspect, desiccant and evaporative cooling is combined with an air scrubber such that spaces can be integrated into the air supply system, in which spaces air is contaminated. Energy in form of thermal energy necessary for the desiccant and evaporative cooling arrangement, for drying the drying agent, may be provided by exhaust heat from other plants on the manufacturing site. The thermal energy could also be provided by solar energy. The air scrubber so-to-speak washes the contaminated air and as a result comprises the particles and/or volatile organic compounds. In case of particles, these can be removed easily via filter technology, whereas for removing the volatile organic compounds from the water leaving the air scrubber, bacteria can be provided such that, as a result, decontaminated and cleaned water is provided that may then be used further in the process of the air supply, or for other purposes.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
- Fig. 1: shows a schematic setup of an air supply system for a manufacturing site according to a first example;
- Fig. 2: shows a schematic setup of an air supply system for a manufacturing site according to a further example;
- Fig. 3: shows a flow diagram of the air supply concept for the example of Fig. 2;
- Fig. 4: shows a schematic setup for a desiccant and evaporative cooling arrangement in Fig. 4A, and a schematic setup of an air supply arrangement in Fig. 4B;
- Fig. 5: shows a further example of a desiccant and evaporative cooling arrangement in a schematic setup;
- Fig. 6: shows a further example of a desiccant and evaporative cooling arrangement;
- Fig. 7: shows a still further example of a desiccant and evaporative cooling arrangement;
- Fig. 8: shows an example of a bioreactor in a schematic setup;
- Fig. 9: shows basic steps of an example for a method for air supply of a manufacturing site;
- Figs. 10 to 13: show further examples for methods for air supply of a manufacturing site;
- Fig. 14: shows a functional diagram of an example of an air supply system for a manufacturing site; and
- Fig. 15: shows steps of a conventional cooling in a Mollier-diagram in Fig. 15A, and the steps of cooling in a desiccant and evaporative cooling arrangement in a Mollier-diagram in Fig. 15B.

### DETAILED DESCRIPTION OF EMBODIMENTS

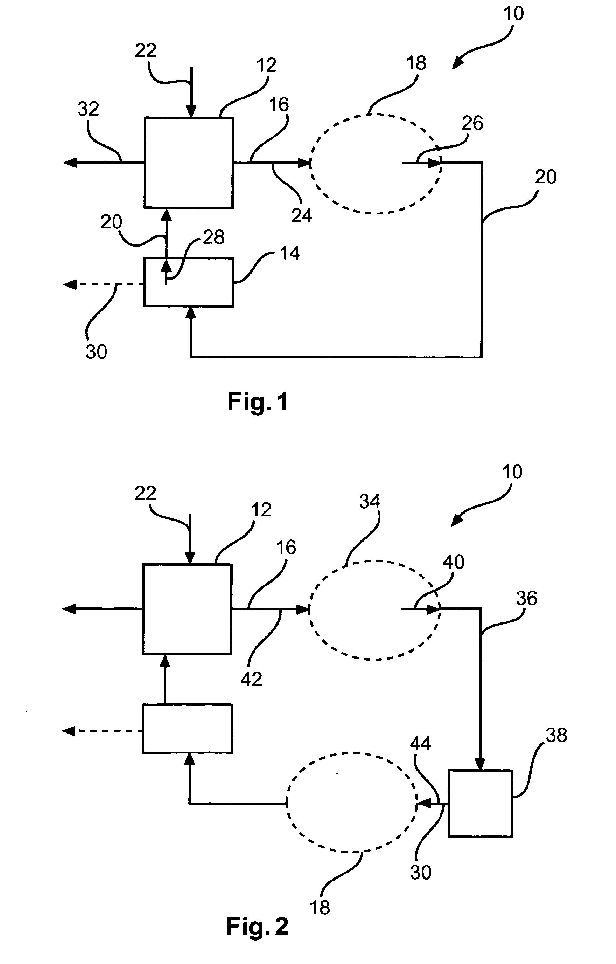

Fig. 1 shows an air supply system 10 for a manufacturing site, comprising a desiccant and evaporative cooling arrangement 12 and an exhaust air scrubber 14. Further, first air supply conduits 16 from the desiccant and evaporative cooling arrangement 12 to at least one contaminated air generating indoor space 18, indicated with a dotted line, are provided, and secondary disposal conduits 20 from the at least one contaminated air generating indoor space 18 to the exhaust air scrubber 14 and to the desiccant and evaporative cooling arrangement 12. The desiccant and evaporative cooling arrangement 12 is configured to condition fresh outdoor air, indicated by arrow 22, as input air for the use in the contaminated air generating indoor space 18. The first air supply conduits 16 are configured to provide the conditioned air as supply air 24 to the at least one contaminated air generating indoor space 18. The secondary disposal conduits 20 are configured to conduct contaminated exhaust air, indicated with reference numeral 26, from the at least one contaminated air generating indoor space 18 to the exhaust air scrubber 14.

The second disposal conduits are further configured to provide the scrubbed air, indicated with arrow 28, to the desiccant and evaporative cooling arrangement 12. The exhaust air scrubber 14 is configured to remove air contamination, indicated with a dotted arrow 30, to a predefined amount, wherein the predefined amount is set such that the air can be used further in the desiccant and evaporative cooling arrangement 12. The scrubbed air is used for at least one of the group of cooling the input air by heat recovery, and desiccating a drying agent of the desiccant and evaporative cooling arrangement 12. An arrow 32 indicates the discharge of air, for example after passing a heat exchange device.

The function of the exhaust cleaning from contamination is based on the waterfall effect, according to which the water drops are polarized by molecular interrelation with the surrounding air; negative charges accumulate on the surface, and positive charge of the drop accumulate in the inside of the drop. Upon hitting a surface, the surface of the drop is destroyed and atomized as small droplets that are sprayed to the air, while the positive charged main drop is floating on the surface. Thus, negative ions are generated which can then be used for cleaning of the exhaust, whereas the water treatment process works according to a biological principle. For example, ceramic structures are used for growing a bio film in which the carriers degrade the volatile organic compounds solved in the water to carbon dioxide. In case the paint shops or the like are not used for a certain period, the bio film can be kept alive via a nutrition solution.

Fig. 2 shows an example of the air supply system 10 for a manufacturing site, comprising different zones for different air conditioning requirements. In addition to the at least one contaminated air generating indoor space 18, at least one air-conditioned indoor space 34 is provided, which is indicated with a dotted line. The first air supply conduits 16 are provided from the desiccant and evaporative cooling arrangement 12 to the at least one air-conditioned indoor space 34. Further, secondary air supply conduits 36 are provided from the at least one air-conditioned indoor space 34 to an air supply arrangement 38, and from the air supply arrangement 38 to the at least one contaminated air generating indoor space 18. The desiccant and evaporative cooling arrangement 12 is configured to condition fresh outdoor air 22 as input air for the use in the air-conditioned indoor space 34. The air supply arrangement 38 is configured to condition exhaust 40 from the at least one air-conditioned indoor space 34 for use in the contaminated air generating space 18. The first air supply conduits 16 are configured to provide the conditioned air as supply air 42 to the at least one air-conditioned indoor space 34, and the secondary air supply conduits 36 are configured to conduct exhaust air 40 from the at least one air-conditioned indoor space 34 to the air supply arrangement 38, and to provide the conditioned air as supply air 44 to the at least one contaminated air generating indoor space 18.

The air-conditioned indoor space 34 may be a fabrication or manufacturing hall, for example for the manufacture of aircrafts, vehicles, trains, coaches, etc. or parts thereof. The contaminated air generating indoor space 18 comprises facilities within manufacturing sites, such as rooms for painting robots, rooms for ovens for curing of paint or other substances, rooms for grinding, filling and polishing, laser welding, or other purposes that generate air contamination.

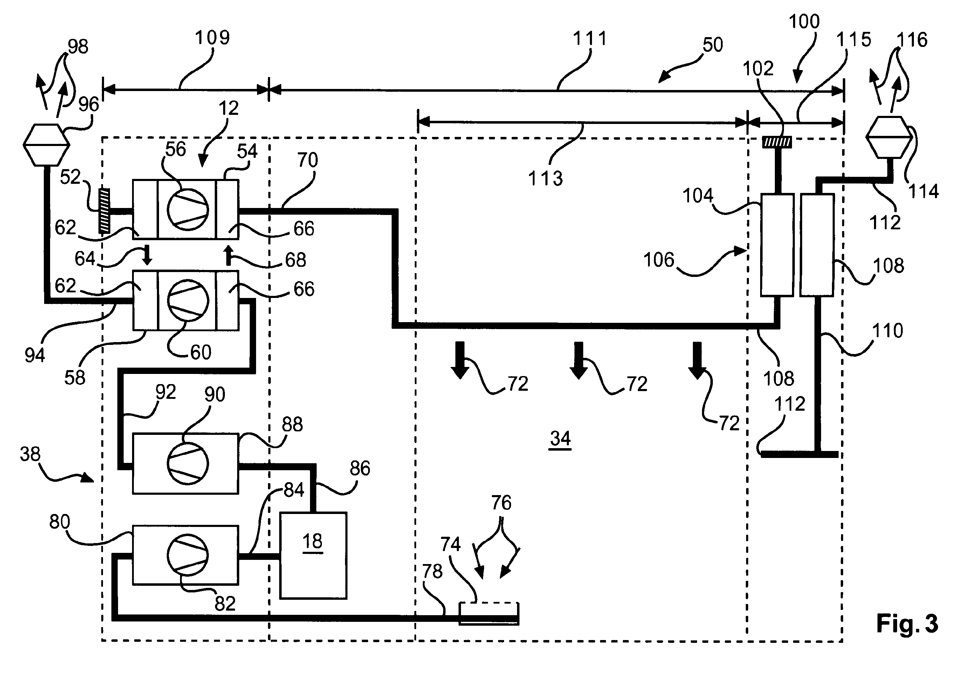

Fig. 3 shows a schematic flow diagram 50. Fresh outdoor air is supplied via air supply 52 and is then conditioned as input air in a supply part 54 of the desiccant and evaporative cooling arrangement 12. The supply part 54 comprises a ventilation device 56 for transporting the air. Below the supply part 54, a discharge part 58 of the desiccant and evaporative cooling arrangement 12 is shown. The discharge part 58 also comprises ventilation means 60. Further, the respective rectangular frame 62 on the left side of the supply part 54 and the discharge part 58 indicates, together with an arrow 64 indicating the transfer of cold from the supply part 54 to the discharge part 58, a desiccant component of the desiccant and evaporative cooling arrangement 12 that, at the same time, also provides a heat exchange. A respective further rectangular frame 66 in the right half of the supply part 54 and the discharge part 58 indicates, once again together with an arrow 68, indicating the transfer of thermal energy, the heat recovery, for example by a heat recovery wheel. An air conduit 70 leaves the supply part 54 to supply air, indicated with arrows 72 to an air-conditioned part of a manufacturing site. Further, a discharge or suction device 74, for example a suction grid arranged in the floor area, is provided for the suction of the exhaust air from the air-conditioned space 34. Arrows 76 indicate the entry of the exhaust air into a supply conduit 78, leading to a supply part 80 of the air supply arrangement 38. The supply part 80 comprises ventilation means 82. The further supply duct 84 leaves the supply part 80 and enters the contaminated air generating indoor space 18, for example a paint cabin. An exhaust discharge duct 86 leaves the contaminated air generating indoor space 18 leading to a discharge part 88 of the supply arrangement 38, also comprising ventilation means 90. A discharge duct 92 leaves the discharge part 88 and leads to the discharge part 58 of the desiccant and evaporative cooling arrangement 12. A discharge or exhaust conduit 94 leaves the discharge part 58 and leads to a discharge opening 96, for example arranged on top of a building. Arrows 98 indicate the discharge of exhaust air. Thus, the desiccant and evaporative cooling arrangement 12 is shown without further indicating the exhaust air scrubber 14, which has been mentioned above.

Further, a pressure-maintaining arrangement 100 is shown in the right half as an option. The pressure-maintaining arrangement 100 comprises a supply duct 102, leading to a first part 104 of a pressure regulation device 106. The first part 104 is connected via a conduit 108 to the supply conduit 70, thus being able to supply air to the indoor space to be air-conditioned in order to increase the air pressure inside the space. Further, a second part 108 of the pressure regulation device 106 is provided, that is connected to a discharge conduit 110 having suction openings 112 arranged in the air-conditioned indoor space 34, and on the other side connected to a discharge conduit 112 leading to a disposal opening 114 to discharge exhaust air 116 in order to decrease the pressure inside the air-conditioned indoor space 34.

A first double arrow 109 indicates an area or part of a building with the function of a gate or lock; a second double arrow 111 indicates an area comprising the hall with a climatic sector, indicated with a third double arrow 113, and an autoclave sector, indicated with a fourth double arrow 115.

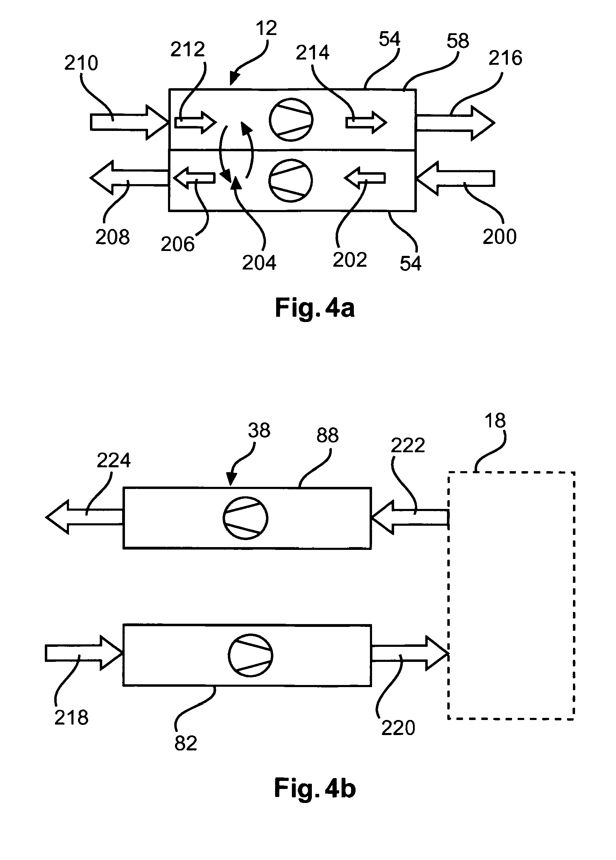

Fig. 4A shows a functional diagram of the desiccant and evaporative cooling arrangement 12. It must be noted that Fig. 4A shows a possible unit, wherein several units can be provided. For example, fresh outdoor air is supplied as indicated with arrow 200, being transported as indicated with arrow 202, and subject to heat/humidity exchange 204, and then further transported, as indicated with arrow 206, to be supplied as supply air 208, for example to the air-conditioned indoor space 34. An arrow 210 indicates the supply of exhaust air from a contaminated air generating indoor space 18, which air is also transported 212 and also subject to the heat/humidity exchange 204, and further transported 214 to leave the system as exhaust air 216.

Fig. 4B shows a functional diagram of the air supply arrangement 38 with the supply part 82 and the discharge part 88. The contaminated air generating indoor space 18 is indicated with a dotted frame. An arrow 218 indicates the entry or supply of exhaust air from the air-conditioned indoor space 34. The air is then treated to have predefined conditions to be supplied to the contaminated air generating indoor space 18 as input air, indicated by arrow 220. An arrow 222 indicates the exhaust air from the contaminated air generating indoor space 18 that is supplied to the desiccant and evaporative cooling arrangement 12, which supply is indicated with further arrow 224.

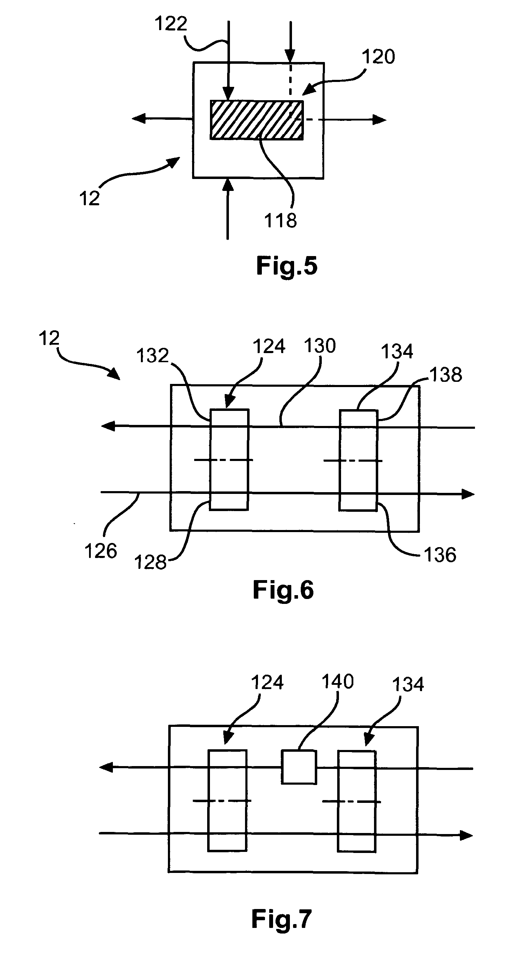

Fig. 5 shows an example where a drying agent 118 is provided in a sorption wheel 120. For example, at least a part of the heat for the cyclic recovery of the drying agent 118 is provided by low thermal energy 122 from a combined heat and power plant (not further shown).

Fig. 6 shows an example of the desiccant and evaporative cooling arrangement 12, comprising at least one dry wheel 124 with a drying agent. The dry wheel 124 is partially arranged within the first air supply duct, indicated with through-going arrow 126, with a first portion 128, and partially arranged within the secondary disposal conduit, indicated with a second through-going arrow 130, with a second portion 132. Further, at least one heat recovery wheel 134 is provided, which is partially arranged within the first air supply duct with a first portion 136, and partially arranged within the secondary disposal conduit with a second portion 138.

According to the example shown in Fig. 7, at least one air heater 140 is provided between the heat recovery wheel 134 and the dry wheel 124 in the secondary disposal conduit.

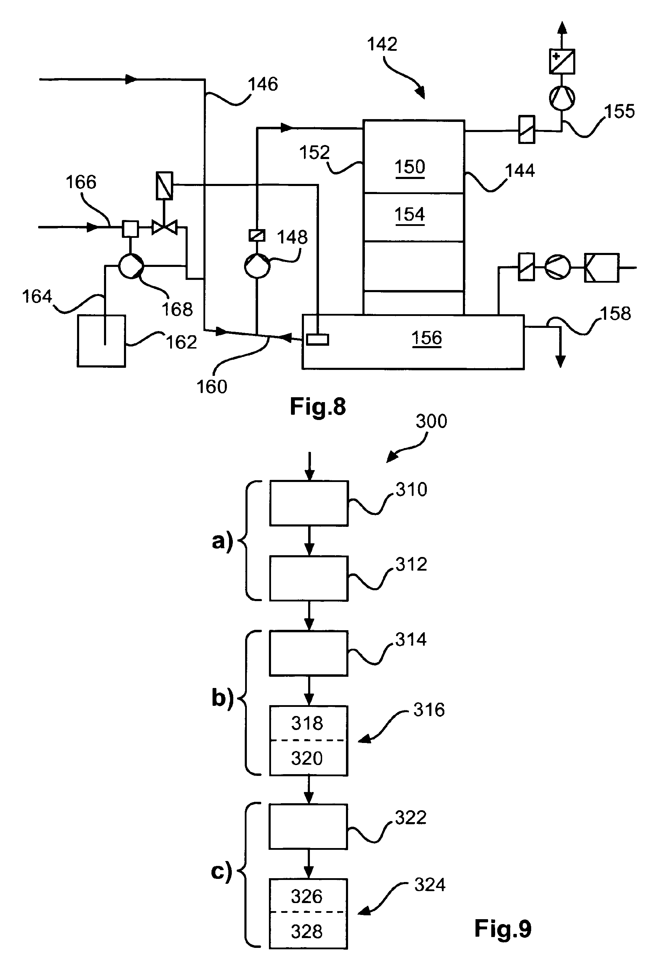

The exhaust air scrubber 14 provides at least one chamber, in which the air conducted through the chamber is subject to spraying of small water droplets with high kinetic energy, which results in the generation of negative ions that are used for cleaning the exhaust air. Thereby, contaminated water is provided that is then supplied to a water treatment arrangement 142 shown in Fig. 8.

As can be seen in Fig. 8, the water treatment arrangement 142 comprises a biological reactor 144, where bacteria provide degrading of the volatile organic compounds into CO₂, for example. Once the volatile organic compounds are removed, for example by a degree of at least 70 %, or for example 90 or even 98 %, the clean waste water can be provided to the desiccant and evaporative cooling arrangement 12, for example for cooling and/or scrubbing purposes in the exhaust air scrubber 14.

Waste water containing contamination such as volatile organic compounds is supplied vie supply line 146. A pump 148 may be provided to provide the transport inside the supply line. The waste water is then passed on to a bio filter unit 150 inside a housing 152 of the biological reactor 144. Below, a base or filler body 154 is provided, followed by a further bio filter layer on a further base or filler body. A ventilation line with a ventilator discharges air, for example above roof level. A reservoir 156 is provided for collecting the cleaned waste water that is now free from volatile compounds. A discharge line 158 is provided, for example in such a height that a minimum amount of cleaned water remains in the reservoir. This water can be used for re-supply to the biological reactor via a supply connection. Further, a second reservoir 162 with a culture medium or nutrition solution is provided connected via supply line 164 to the supply line 146. As a pump, drinking water may be provided via line 166 and a valve, such that drinking water mixed with nutrition solution is supplied to supply line 146 and to the bio filter 150 in one scenario. In another scenario, in addition or alternative to the one mentioned above, a filling level monitoring device monitors the level inside the reservoir 156; in case of sufficient water in the reservoir 156, in addition to or instead of the drinking water, the water in the reservoir 156 can be used together with the nutrition solution from the second reservoir to keep the bacteria of the biological reactor alive in periods where no or only very little waste water accumulates.

Further, a method 300 for air supply of a manufacturing site is shown in Fig. 9, comprising the following steps: in a first step a), a first sub-step 310 is provided in which fresh outdoor air is conditioned as input air in a desiccant and evaporative cooling arrangement for the use in contaminated air generating indoor spaces. As a second sub-step 312, the conditioned air is provided as supply air to at least one contaminated air generating indoor space. In a step b), as a third sub-step 314, contaminated exhaust air is conducted from the at least one contaminated air generating indoor space to an exhaust air scrubber, where, in a fourth sub-step 316, water is sprayed to humidify and cool 318 the air, and to remove 320 air contamination to a predefined amount, wherein the predefined amount is set such that the air can be used further in the desiccant and evaporative cooling arrangement. Further, in a step c), as a fifth sub-step 322, the scrubbed air is provided to the desiccant and evaporative cooling arrangement, wherein the scrubbed air is used in a sixth sub-step 324 for at least one of the group of cooling 326 the input air by heat recovery, and desiccating 328 a drying agent of the desiccant and evaporative cooling arrangement.

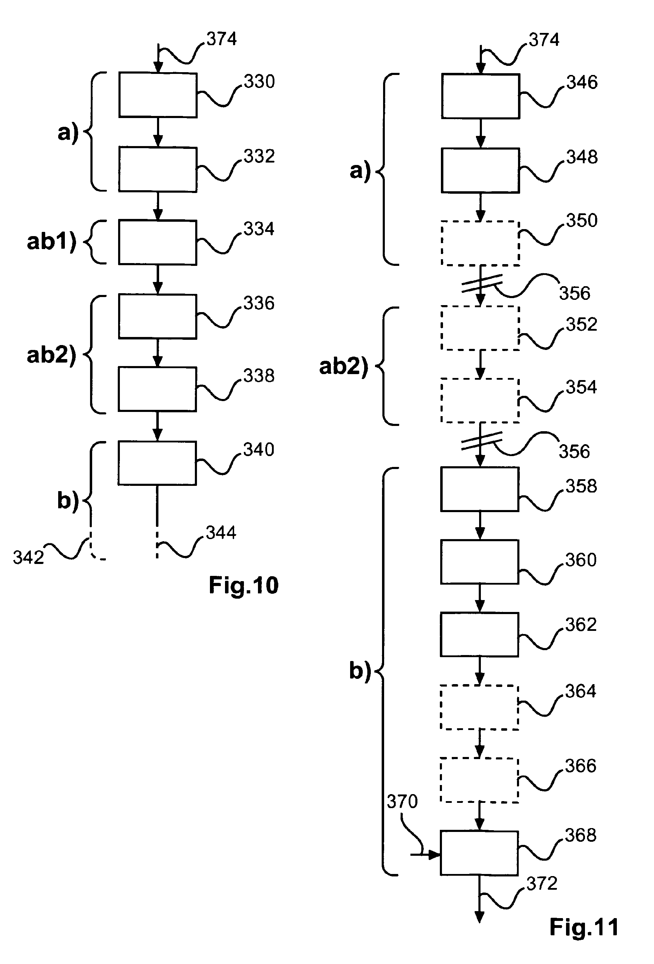

According to the example shown in Fig. 10, the manufacturing site is comprising different zones with different air-conditioned requirements, and in step a), the fresh outdoor air is conditioned in a sub-step 330 for the use in air-conditioned indoor spaces, and the conditioned air is provided in a further sub-step 332 as supply air to at least one air-conditioned indoor space. Further, between step a) and step b), a step ab1) is provided, in which exhaust air is conducted in a sub-step 334 from the at least one air-conditioned indoor space to an air supply arrangement of contaminated air generating spaces. In a further step ab2), exhaust air in the air supply arrangement is conditioned in a further sub-step 336 for the use in contaminated air generating spaces, and in a still further sub-step 338, the conditioned exhaust air is provided as the supply air to the at least one contaminated air generating indoor space. Further, in step b), a sub-step 340 is provided, in which contaminated exhaust air from the at least one contaminated air generating indoor space is conducted to the exhaust air scrubber. Further, a dotted end of the bracket, indicated with reference numeral 342, and a dotted end of the lines following frame 340, indicated with a dotted line 344, indicate that following, further steps are provided, for example, as described above.

Fig. 11 shows a further example, in which step a) comprises as a sub-step 346 of drying of the input air and simultaneous heating of the input air. As a still further sub-step 348, cooling the heated input air by a heat recovery wheel and humidifying of the input air is provided. Following, three optional sub-steps, indicated with dotted frames, are provided. In an optional sub-step 350 of step a) additional heating is provided. Further, step ab2) comprises a first optional sub-step 352 of optionally humidifying, and a second optionally sub-step 352 of optionally additional heating. It must be noted that two inclined parallel lines 356 indicate that further steps may be provided in-between.

Still further, step b) comprises a first sub-step 358, in which humidifying and simultaneous scrubbing by high speed spraying of water onto a surface is provided, where the water drops are atomized into smaller droplets generating negative charges. In a still further sub-step 360, absorption of thermal energy from the heat recovery wheel is provided by the air cooled by the humidification. Thereby, as a still further sub-step 362, heating up the air is provided. This is followed by two further optional sub-steps. In an optional sub-step 364, optional heating of the air by low temperature moisture expeller is provided. As a further sub-step 366, optional heating of the air by high temperature moisture expeller for disinfection is provided. Finally, a further sub-step 368 is provided in which the drying agent is dried by hot air, which hot air is indicated with an arrow 370, entering the frame 368. Further, a downward pointing arrow 372 indicates that further steps may be provided; an upper arrow 374 indicates the provision of fresh air as the supply air.

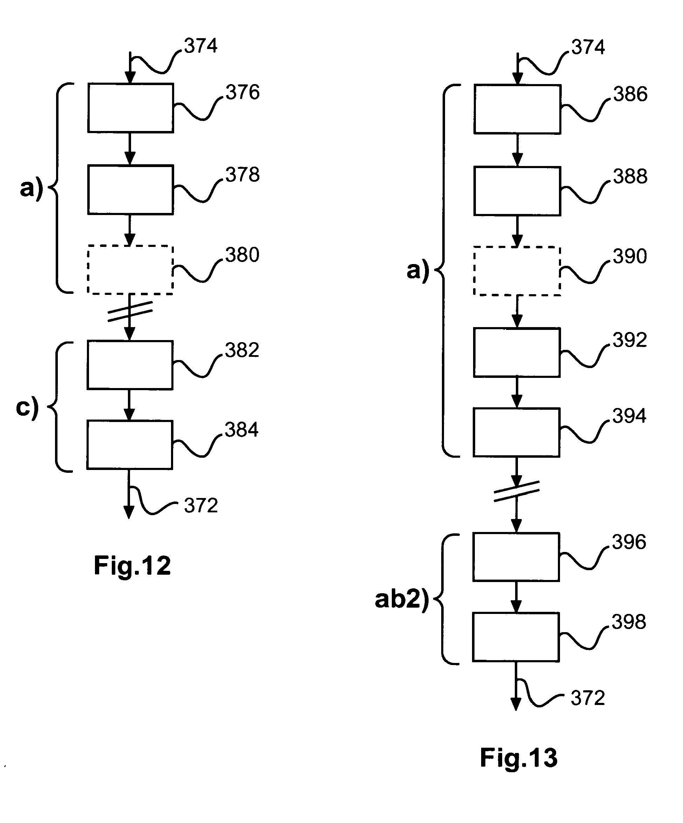

Fig. 12 shows an example in which step a) comprises a sub-step 376 of first humidifying a pre-heating of the input air with an absorption wheel. As a further sub-step 378, a heating with a high temperature heater is provided. As an optional sub-step 380, heating with a cooler that also works as a low temperature heater, in relation with the scrubber is provided. Further, a step ab2) comprises a sub-step 382 of increasing of the air temperature by recirculation cooler/heater heating, and a further sub-step 384 of second humidifying.

Fig. 13 shows an example, according to which step a) comprises as a sub-step 386 of first humidifying and pre-heating of the input air with an absorption wheel. As a further sub-step 388, heating with a high temperature heater is provided. This is followed by an optional sub-step 390, of optionally heating (indicated with dotted frame) with a cooler that also works as a low temperature heater, in relation with scrubber. Next, a sub-step 392 of humidifying and cooling with input humidifier is provided. This is followed by a sub-step 394 of heating with cooler that also works as low temperature heater. Further, step ab2) comprises a sub-step 396 of increasing of the air temperature by recirculation cooler/heater heating; and a further sub-step 398 of second humidifying.

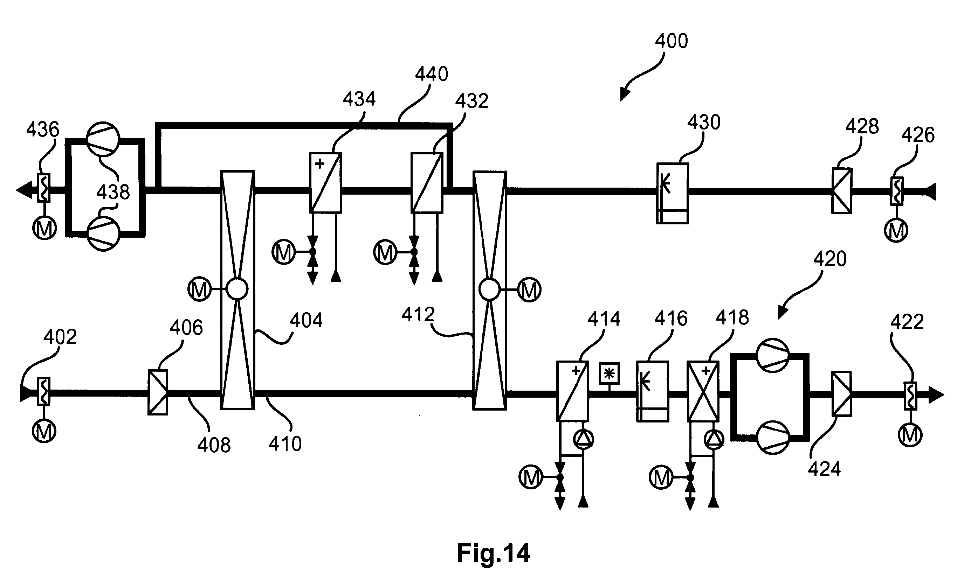

Fig. 14 shows a flow diagram 400 with functional components/units in an overview. Air enters at 402 as fresh air from the outside of a building, and is supplied to a dryer 404. For the air transport, compression or suction means, i.e. transporting means, are provided. Further, a filter arrangement 406 may be provided. While drying the input air in the dryer 404, the air is simultaneously heated, which is indicated with a change of the colour or pattern of supply line 408 compared to supply line 410 on the other side of the dryer 404. Further, a heat recovery wheel 412 for cooling is provided. In the following, a first optional additional conditioner 414 (functioning as a heater in winter and as a cooler in summer) 414 is provided, followed an optional humidifying unit 416, and further followed by a second optional thermal conditioner 418, i.e. additional cooler in the summer scenario and an additional heating in the winter scenario. The air is supplied to a usable space to be supplied with air following the first additional heater 414. Following the second additional heater 418, a ventilator unit 420 is provided. The air can then be discharged via a supply outlet 422 to the air-conditioned space, i.e. the climatic zone, for example with the support of a further ventilation or compression device 424. In the climatic zone the air is contaminated, for example with VOCs, such as in contaminated air generating spaces. In the upper part of the flow diagram, an exhaust discharge opening 426 for sucking the exhaust air, for example contaminated with volatile organic compounds, for example further followed by further compression means. Still further, a second filter arrangement 428 is provided. Next, a scrubbing unit 430 is provided for humidifying and simultaneous scrubbing the air by high speed spraying of water; and the thus scrubbed air is then supplied to the heat recovery wheel 412. Further, a first optional low thermal heater 432 may be provided, and may also be followed by a high temperature heater 434. The air is then further supplied to the dryer 404, i.e. a dry wheel, before being discharged via discharge opening 436 with the aid of, for example, two parallel compression/ventilation units 438. Further, instead of being supplied to the low thermal heater 432, the high thermal heater 434, and the dryer 404 or dry wheel, a bypass 440 is provided.

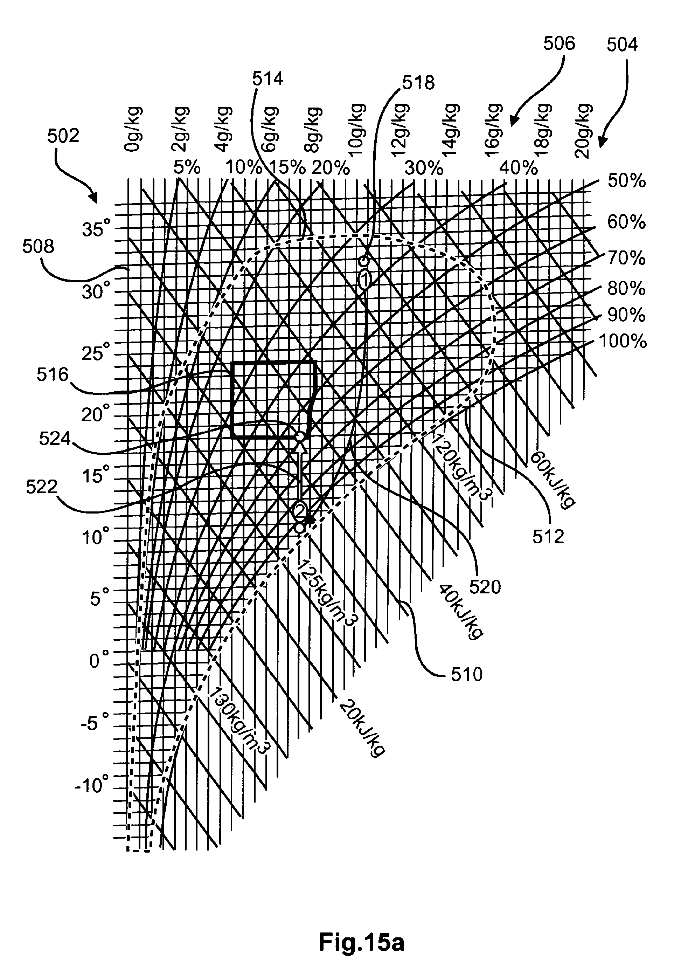

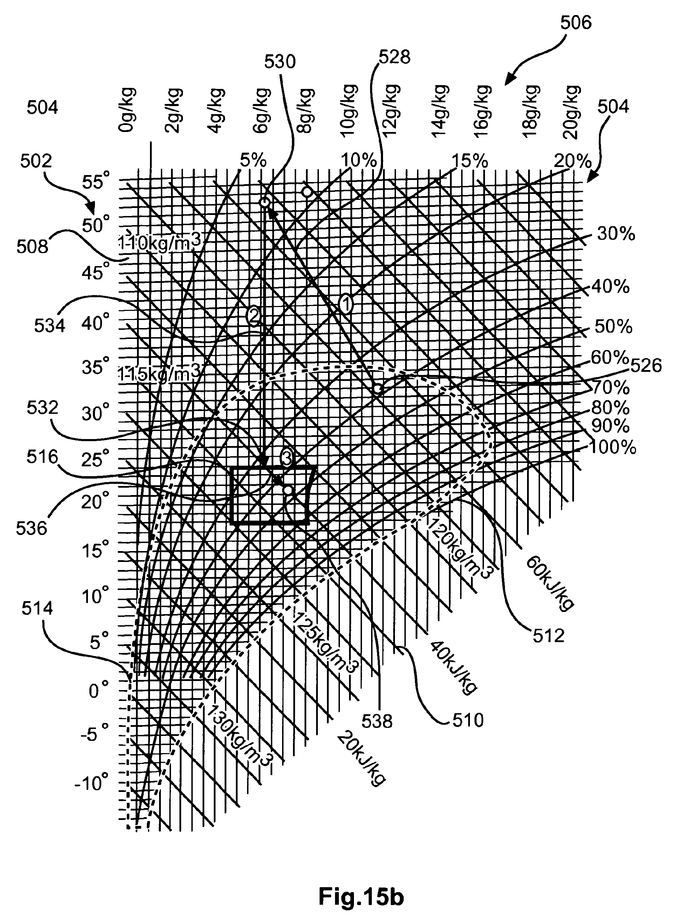

Fig. 15A and Fig. 15B show a Mollier-h-x-diagram for air with rather large humidity and a pressure of 1.012 bar. In the diagram, a vertical left scale 502 indicates the temperature, whereas a vertical scale on the right, indicated with 504, shows the relative humidity. In the upper horizontal scale, water in gram per kilogram is indicated with a horizontal scale 506. A background grid 508 is provided, together with first line structure 510, indicating lines with similar enthalpy, and second type of lines 512, indicating similar relative humidity. Further, a first background pattern enclosed by dotted line 514 indicates meteorological data of Hamburg. An area 516 filled with a second pattern indicates a range of acceptable values for the temperature, the values for water in gram per kilogram, and a relative humidity. In conventional cooling, shown in Fig. 15A, for example summer air having parameters at point 518 of approximately 32 °C with a relative humidity of approximately 35 % is subject to cooling in first step 520, whereby the relative humidity may reach the absorption point of 100 % relative humidity. In a second step 522, the air is then heated up to reach a second point 524 that lies within the acceptable range. Fig. 15B shows the respective situation for cooling in a desiccant and evaporative cooling arrangement. Starting from a first point 526, with, for example, a temperature of 32 °C and a relative humidity of about 38 %, the air is supplied to a drier in a first step 528, reaching intermediate first point 530, for example an air temperature of 52 °C and a relative humidity of about 7.5 %. The air is then subject to heat recovery, leading to a second intermediate point or state 532, for example a temperature of 24 °C and a relative humidity of 32 %. The heat recovery is thus a second step 534. In a third step 536, the air is humidified and cooled simultaneously, leading to a final state 538 lying within the acceptable range 516.

As indicated above, it is possible to reuse the exhaust air from the air-conditioned indoor space in the contaminated air generating indoor space(s). For example, only a humidification may be necessary, thus omitting additional energy costs for heating or cooling. After decontaminating the exhaust air, the exhaust air is supplied to the desiccant and evaporative cooling arrangement for making use of the energy, i.e. the thermal energy of the exhaust air. Further, the use of discharged heat of a combined heat and power plant in a range of low temperature further increases the energy efficiency of the above-described air supply system for a manufacturing site.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features. While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims. In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An air supply system (10) for a manufacturing site, comprising:
- a desiccant and evaporative cooling arrangement (12);
- an exhaust air scrubber (14);
- first air supply conduits (16) from the desiccant and evaporative cooling arrangement to at least one contaminated air generating indoor space (18);
- secondary disposal conduits (20) from the at least one contaminated air generating indoor space to the exhaust air scrubber and to the desiccant and evaporative cooling arrangement;
wherein the desiccant and evaporative cooling arrangement is configured to condition fresh outdoor air (22) as input air for the use in the contaminated air generating indoor space;
wherein the first air supply conduits are configured to provide the conditioned air as supply air (24) to the at least one contaminated air generating indoor space; and the secondary disposal conduits are configured to conduct contaminated exhaust air (26) from the at least one contaminated air generating indoor space to the exhaust air scrubber; and to provide the scrubbed air to the desiccant and evaporative cooling arrangement; and
wherein the exhaust air scrubber is configured to remove air contamination (30) to a predefined amount, wherein the predefined amount is set such that the air can be used further in the desiccant and evaporative cooling arrangement; wherein it is provided to remove volatile organic compounds by a degree of at least approximately 70%; and
wherein the scrubbed air is used for at least one of the group of:
- cooling the input air by heat recovery; and
- desiccating a drying agent of the desiccant and evaporative cooling arrangement; and **characterized in that**
the first air supply conduits are provided from the desiccant and evaporative cooling arrangement to the at least one air-conditioned indoor space;
secondary air supply conduits (36) are provided from the at least one air-conditioned indoor space to an air supply arrangement (38), and from the air supply arrangement to the at least one contaminated air generating indoor space;
the desiccant and evaporative cooling arrangement is configured to condition fresh outdoor air as input air for the use in the air-conditioned indoor space;
the air supply arrangement is configured to condition exhaust air (40) from the at least one air-conditioned indoor space for the use in the contaminated air generating space; and
the first air supply conduits are configured to provide the conditioned air as supply air to the at least one air-conditioned indoor space; and the secondary air supply conduits are configured to conduct exhaust air (40) from the at least one air-conditioned indoor space to the air supply arrangement; and to provide the conditioned air as supply air (44) to the at least one contaminated air generating indoor space.

2. System according to claim 1, wherein it is provided
- at least one air-conditioned indoor space; and/or
- at least one contaminated air generating indoor space.

3. System according to one of the preceding claims, wherein the desiccant and evaporative cooling arrangement comprises a drying agent (118) provided in a sorption-generator (120); and at least a part of the heat for the cyclic recovery of the drying agent is provided by low thermal energy (122) from a combined heat and power plant.

4. System according to one of the preceding claims, wherein the desiccant and evaporative cooling arrangement comprises:
- at least one dry wheel (124) with a drying agent, which dry wheel is arranged within the first air supply duct with a first portion (128) and within the secondary disposal conduit with a second portion (132); and
- at least one heat recovery wheel (134), which is arranged within the first air supply duct with a first portion (136) and within the secondary disposal conduit with a second portion (138).

5. System according to one of the preceding claims, wherein at least one air heater (140) is provided between the heat recovery wheel and the dry wheel in the secondary disposal conduit.

6. System according to one of the preceding claims, wherein the exhaust air scrubber is an integrated unit of the desiccant and evaporative cooling arrangement.

7. A method (300) for air supply of a manufacturing site, comprising the following steps:
a) conditioning (310) of fresh outdoor air in a desiccant and evaporative cooling arrangement as input air for the use in the contaminated air generating indoor space; and providing (312) the conditioned air as supply air to at least one contaminated air generating indoor space;
b) conducting (314) of contaminated exhaust air from the at least one contaminated air generating indoor space to an exhaust air scrubber where water is sprayed to i) humidify and cool (318) the air, and to ii) remove (320) air contamination to a predefined amount, wherein the predefined amount is set such that the air can be used further in the desiccant and evaporative cooling arrangement; wherein it is provided to remove volatile organic compounds by a degree of at least approximately 70%; and
c) providing (322) the scrubbed air to the desiccant and evaporative cooling arrangement; wherein the scrubbed air is used for at least one of the group of:
- cooling (326) the input air by heat recovery; and
- desiccating (328) a drying agent of the desiccant and evaporative cooling arrangement;
wherein **characterized in that** the manufacturing site is comprising different zones with different air conditioning requirements;
in step a) the fresh outdoor air is conditioned (330) for the use in air-conditioned indoor spaces, and the conditioned air is provided (332) as supply air to at least one air-conditioned indoor space;
between step a) and step b) these steps are provided:
ab1) conducting (334) of exhaust air from the at least one air-conditioned indoor space to an air supply arrangement of contaminated air generating spaces; and
ab2) conditioning (336) of the exhaust air in the air supply arrangement for the use in contaminated air generating spaces; and providing (338) the conditioned air as the supply air to the at least one contaminated air generating indoor space; and
in step b) contaminated exhaust air from the at least one contaminated air generating indoor space is conducted (340) to the exhaust air scrubber.

8. Method according to claim 7, wherein the exhaust air from the air-conditioned indoor spaces is used as air source for contaminated air generating spaces; and
wherein the exhaust air from the contaminated air generating indoor spaces and/or for the air-conditioned indoor spaces is used for operating the desiccant and evaporative cooling arrangement for conditioning the supply air for the contaminated air generating indoor space and/or air-conditioned indoor spaces.

9. Method according to claim 7 or 8, wherein the desiccant and evaporative cooling arrangement comprises a drying agent provided in a sorption-generator; and the cyclic recovery of the drying agent is supported by low thermal energy from a combined heat and power plant.

10. Method according to one of the claims 7 to 9, wherein the air contamination is provided by:
i) particles, wherein the amount of sprayed water that is not solved in the air is at least partly collected and supplied to a filter arrangement; and/or
ii) volatile organic compounds, wherein the amount of sprayed water that is not solved in the air is at least partly collected and supplied to a biological reactor, where bacteria provide degrading of the volatile organic compounds into CO₂.

11. Method according to one of the claims 7 to 10,
wherein step a) comprises:
- drying (346) of the input air and simultaneous heating of the input air;
- cooling (348) of the heated input air by a heat recovery wheel and humidifying of the input air;
- optionally additional heating (350);
wherein step ab2) comprises:
- optionally humidifying (352);
- optionally additional heating (354); and
wherein step b) comprises:
- humidifying and simultaneous scrubbing (358) by high speed spraying of water onto a surface where the water drops are atomized into smaller droplets generating negative charges;
- absorption (360) of thermal energy from the heat recovery wheel by the air cooled by the humidification; and thereby heating (362) up the air;
- optionally heating (364) of the air by low temperature moisture expeller;
- optionally heating (366) of the air by high temperature moisture expeller for disinfection; and
- drying (368) of the drying agent by hot air.

12. Method according to one of the claims 7 to 11,
wherein step a) comprises:
- first humidifying and pre-heating (376) of the input air with an absorption wheel;
- heating (378) with a high temperature heater;
- optionally heating (380) with a cooler that also works as a low temperature heater, in relation with the scrubber; and
wherein step ab2) comprises:
- increasing (382) of the air temperature by recirculation cooler/heater heating; and
- second humidifying (384).

13. Method according to one of the claims 7 to 12,
wherein step a) comprises:
- first humidifying and pre-heating (386) of the input air with an absorption wheel;
- heating (388) with a high temperature heater;
- optionally heating (390) with a cooler that also works as a low temperature heater, in relation with scrubber;
- humidifying and cooling (392) with input humidifier;
- heating (394) with cooler that also works as low temperature heater; and
wherein step ab2) comprises:
- increasing (396) of the air temperature by recirculation cooler/heater heating; and
- second humidifying (398).

## Patentansprüche

1. Ein Luftversorgungssystem (10) für eine Fertigungsstätte, aufweisend:
- eine Vorrichtung zur Kühlung durch Trocknung und Verdunstung (12);
- einen Abluftwäscher (14);
- erste Luftversorgungsleitungen (16) von der Vorrichtung zur Kühlung durch Trocknung und Verdunstung zu wenigstens einem verunreinigte Luft erzeugenden Innenraum (18);
- sekundäre Abluftleitungen (20) von dem wenigstens einen Innenraum, in dem verunreinigte Luft erzeugt wird, zum Abluftwäscher und zur Vorrichtung zur Kühlung durch Trocknung und Verdunstung;
wobei die Vorrichtung zur Kühlung durch Trocknung und Verdunstung ausgeführt ist, frische Außenluft (22) aufzubereiten als Eingangsluft zum Gebrauch in dem Innenraum, in dem verunreinigte Luft entsteht;
wobei die ersten Luftversorgungsleitungen ausgeführt sind, die aufbereitete Luft als Zuluft (24) bereitzustellen zu dem wenigstens einen Innenraum, in dem verunreinigte Luft entsteht; und die zweiten Abluftleitungen ausgeführt sind, verunreinigte Abluft (26) von dem wenigstens einen Innenraum, in dem verunreinigte Luft entsteht, zum Abluftwäscher zu führen; und die gewaschene Luft der Vorrichtung zur Kühlung durch Trocknung und Verdunstung zur Verfügung zu stellen; und
wobei der Abluftwäscher ausgeführt ist, Luftverunreinigung (30) um eine vordefinierte Menge zu entfernen, wobei die vordefinierte Menge so gewählt ist, dass die Luft weiterhin in der Vorrichtung zur Kühlung durch Trocknung und Verdunstung verwendet werden kann; wobei es vorgesehen ist, flüchtige organische Verbindungen um einen Anteil von mindestens näherungsweise 70 % zu entfernen; und
wobei die gewaschene Luft verwendet wird für mindestens eines aus der Gruppe von:
- Kühlen der Eingangsluft durch Wärmerückgewinnung; und
- Trocknen eines Trockenmittels der Vorrichtung zur Kühlung durch Trocknung und Verdunstung; und
**dadurch gekennzeichnet, dass**
die ersten Luftversorgungsleitungen vorgesehen sind von der Vorrichtung zur Kühlung durch Trocknung und Verdunstung zu dem wenigstens einen klimatisierten Innenraum;
sekundäre Luftversorgungsleitungen (36) vorgesehen sind von dem wenigstens einen klimatisierten Innenraum zu einer Luftversorgungsvorrichtung (38) und von der Luftversorgungseinrichtung zu dem wenigstens einen Innenraum, in dem verunreinigte Luft entsteht;
die Vorrichtung zur Kühlung durch Trocknung und Verdunstung ausgeführt ist, frische Außenluft als Eingangsluft aufzubereiten zur Verwendung in dem klimatisierten Innenraum;
die Luftversorgungseinrichtung ausgeführt ist, Abluft (40) von dem wenigstens einen klimatisierten Innenraum aufzubereiten zur Benutzung in dem Raum, in dem verunreinigte Luft entsteht; und
die ersten Luftversorgungsleitungen ausgeführt sind, die aufbereitete Luft als Zuluft zu dem wenigstens einen klimatisierten Innenraum zur Verfügung zu stellen; und die sekundären Luftversorgungsleitungen ausgeführt sind, Abluft (40) von dem wenigstens einen klimatisierten Innenraum zu der Luftversorgungseinrichtung zu führen; und die aufbereitete Luft als Zuluft (44) zur Verfügung zu stellen zu dem wenigstens einen Innenraum, in dem verunreinigte Luft entsteht.

2. System nach Anspruch 1, wobei vorgesehen ist
- wenigstens ein klimatisierter Innenraum; und/oder
- wenigstens ein Innenraum, in dem verunreinigte Luft entsteht.

3. System nach einem der vorhergehenden Ansprüche, wobei die Vorrichtung zur Kühlung durch Trocknung und Verdunstung ein Trockenmittel (118) aufweist, das in einem Sorptionsgenerator (120) vorgesehen ist; und wenigstens ein Teil der Wärme für die zyklische Rückgewinnung des Trockenmittels durch niedrige thermische Energie (122) von einer Kraft-Wärme-Kopplung Anlage zur Verfügung gestellt wird.

4. System nach einem der vorhergehenden Ansprüche, wobei die Vorrichtung zur Kühlung durch Trocknung und Verdunstung aufweist:
- wenigstens einen Rotationsentfeuchter (124) mit einem Trockenmittel, wobei der Rotationsentfeuchter innerhalb der ersten Zuluftzuführung mit einem ersten Bereich (128) und innerhalb der sekundären Abluftleitung mit einem zweiten Bereich (132) angeordnet ist; und
- wenigstens einen Rotationswärmetauscher (134), der innerhalb der ersten Zuluftzuführung mit einem ersten Bereich (136) und innerhalb der sekundären Abluftleitung mit einem zweiten Bereich (138) angeordnet ist.

5. System nach einem der vorhergehenden Ansprüche, wobei wenigstens ein Lufterwärmer (140) zwischen dem Rotationswärmetauscher und dem Rotationsentfeuchter in der sekundären Abluftleitung vorgesehen ist.

6. System nach einem der vorhergehenden Ansprüche, wobei der Abluftwäscher eine integrierte Einheit der Vorrichtung zur Kühlung durch Trocknung und Verdunstung ist.

7. Ein Verfahren (300) für die Luftversorgung einer Fertigungsstätte, aufweisend die folgenden Schritte:
a) Aufbereiten (310) von frischer Außenluft in einer Vorrichtung zur Kühlung durch Trocknung und Verdunstung als Eingangsluft zur Verwendung in einem Innenraum, in dem verunreinigte Luft entsteht; und Zurverfügungstellen (312) der aufbereiteten Luft als Zuluft zu wenigstens einem Innenraum, in dem verunreinigte Luft entsteht;
b) Leiten (314) von verunreinigter Abluft von dem wenigstens einen Innenraum, in dem verunreinigte Luft entsteht, zu einem Abluftwäscher, wo Wasser eingespritzt wird, um i) die Luft zu befeuchten und zu kühlen (318), und um ii) Luftverunreinigung um eine vordefinierte Menge zu entfernen (320), wobei die vordefinierte Menge so gewählt ist, dass die Luft weiterhin in der Vorrichtung zur Kühlung durch Trocknung und Verdunstung verwendet werden kann; wobei es vorgesehen ist, flüchtige organische Verbindungen um einen Betrag von mindestens näherungsweise 70 % zu entfernen; und
c) Zurverfügungstellen (322) der gewaschenen Luft zur Vorrichtung zur Kühlung durch Trocknung und Verdunstung; wobei die gewaschene Luft für wenigstens eines aus der folgenden Gruppe verwendet wird:
- Kühlen (326) der Eingangsluft durch Wärmerückgewinnung; und
- Trocknen (328) eines Trockenmittels der Vorrichtung zur Kühlung durch Trocknung und Verdunstung;
**dadurch gekennzeichnet, dass**
die Fertigungsstätte verschiedene Zonen mit verschiedenen Anforderungen an die Klimatisierung aufweist;
in Schritt a) die frische Außenluft aufbereitet wird (330) zur Verwendung in klimatisierten Innenräumen, und die aufbereitete Luft als Zuluft zur Verfügung gestellt wird (332) für wenigstens einen klimatisierten Innenraum;
zwischen Schritt a) und Schritt b) folgende Schritte vorgesehen sind:
ab1) Leiten (334) von Abluft von dem wenigstens einen klimatisierten Innenraum zu einer Luftversorgungseinrichtung von Räumen, in denen verunreinigte Luft entsteht; und
ab2) Aufbereiten (336) der Abluft in der Luftversorgungseinrichtung zur Benutzung in Räumen, in denen verunreinigte Luft entsteht; und Zurverfügungstellen (338) von aufbereiteter Luft als Zuluft zu dem wenigstens einen Innenraum, in dem verunreinigte Luft entsteht; und
in Schritt b) verunreinigte Abluft von dem wenigstens einen Innenraum, in dem verunreinigte Luft entsteht, zum Abluftwäscher geführt wird (340).

8. Verfahren nach Anspruch 7, wobei die Abluft der klimatisierten Innenräume als Luftquelle für Räume, in denen verunreinigte Luft entsteht, verwendet wird; und
wobei die Abluft von den Innenräumen, in denen verunreinigte Luft entsteht, und/oder für die klimatisierten Innenräume verwendet wird, um die Vorrichtung zur Kühlung durch Trocknung und Verdunstung zu betreiben für die Aufbereitung der Zuluft für die Innenräume, in denen verunreinigte Luft entsteht, und/oder klimatisierten Innenräume.

9. Verfahren nach Anspruch 7 oder 8, wobei die Vorrichtung zur Kühlung durch Trocknung und Verdunstung ein Trockenmittel aufweist, das in einem Sorptionsgenerator vorgesehen ist; und die zyklische Wiedergewinnung des Trockenmittels von niedriger thermischer Energie von einer Kraft-Wärme-Kopplung Anlage unterstützt wird.

10. Verfahren nach einem der Ansprüche 7 bis 9, wobei die Luftverunreinigung verursacht wird durch:
i) Partikel, wobei die Menge des eingespritzten Wassers, das nicht in der Luft gelöst wird, wenigstens teilweise gesammelt und in eine Filteranlage gespeist wird; und/oder
ii) flüchtige organische Verbindungen, wobei die Menge des eingespritzten Wassers, das nicht in der Luft gelöst wird, wenigstens teilweise gesammelt und an einen Bioreaktor gespeist wird, wobei Bakterien den Abbau der flüchtigen organischen Verbindungen in CO₂ besorgen.

11. Verfahren nach einem der Ansprüche 7 bis 10,
wobei Schritt a) aufweist:
- Trocknen (346) der Eingangsluft und gleichzeitiges Erwärmen der Eingangsluft;
- Kühlen (348) der erwärmten Eingangsluft durch einen Rotationswärmetauscher und Befeuchten der Eingangsluft;
- optionales zusätzliches Erwärmen (350);
wobei Schritt ab2) umfasst:
- optionales Befeuchten (352);
- optionales zusätzliches Erwärmen (354); und
wobei Schritt b) umfasst:
- Befeuchten und gleichzeitiges Waschen (358) durch Hochgeschwindigkeitseinspritzen von Wasser auf eine Oberfläche, wo die Wassertropfen in kleinere Tröpfchen zerstäubt werden, was negative Ladungen generiert;
- Absorption (360) thermischer Energie des Rotationswärmetauschers durch die Luft, die durch die Befeuchtung gekühlt wird; und dabei ein Aufwärmen (362) der Luft;
- optionales Erwärmen (364) der Luft durch Niedrigtemperaturfeuchtigkeitsentzieher;
- optionales Erwärmen (366) der Luft durch Hochtemperaturfeuchtigkeitsentzieher zur Desinfektion; und
- Trocknen (368) des Trockenmittels durch heiße Luft.

12. Verfahren nach einem der Ansprüche 7 bis 11,
wobei Schritt a) aufweist:
- erstes Befeuchten und Vorwärmen (376) der Eingangsluft mit einem Rotationsabsorber;
- Erwärmen (378) mit einem Hochtemperaturerwärmer;
- optionales Erwärmen (3 80) mit einem Kühler, der auch als Niedrigtemperaturerwärmer funktioniert, in Verbindung mit dem Wäscher, und wobei Schritt ab2) aufweist:
- Erhöhen (382) der Lufttemperatur durch Rezirkulation der Kühler-/Heizwärme; und
- zweites Befeuchten (384).

13. Verfahren nach einem der Ansprüche 7 bis 12,
wobei Schritt a) umfasst:
- erstes Befeuchten und Vorwärmen (386) der Eingangsluft mit einem Rotationsabsorber;
- Erwärmen (388) mit einem Hochtemperaturerwärmer;
- optionales Erwärmen (390) mit einem Kühler, der auch als Niedrigtemperaturheizung funktioniert, in Verbindung mit einem Wäscher;
- Befeuchten und Kühlen (392) mit einem Eingangsbefeuchter;
- Erwärmen (394) mit einem Kühler, der auch als Niedrigtemperaturheizung funktioniert; und
wobei Schritt ab2) umfasst:
- Erhöhen (396) der Lufttemperatur durch Rezirkulation der Kühler-/Heizungswärme; und
- zweites Befeuchten (398).

## Revendications

1. Système d'alimentation en air (10) pour un site de production, comportant :
- un agencement de dessiccation et de refroidissement par évaporation (12),
- un épurateur d'air évacué (14),
- des premières conduites d'alimentation en air (16) allant de l'agencement de dessiccation et de refroidissement par évaporation jusqu'à au moins un espace intérieur générant de l'air contaminé (18),
- des conduites secondaires de rejet (20) allant du au moins un espace intérieur générant de l'air contaminé jusqu'à l'épurateur d'air évacué et jusqu'à l'agencement de dessiccation et de refroidissement par évaporation,
dans lequel l'agencement de dessiccation et de refroidissement par évaporation est configuré pour conditionner de l'air extérieur frais (22) en tant qu'air d'entrée en vue de l'utilisation dans l'espace intérieur générant de l'air contaminé,
dans lequel les premières conduites d'alimentation en air sont configurées pour fournir l'air conditionné en tant qu'air d'alimentation (24) au au moins un espace intérieur générant de l'air contaminé, et les conduites secondaires de rejet sont configurées pour diriger l'air évacué contaminé (26) depuis le au moins un espace intérieur générant de l'air contaminé jusqu'à l'épurateur d'air évacué, et pour fournir l'air épuré à l'agencement de dessiccation et de refroidissement par évaporation, et
dans lequel l'épurateur d'air évacué est configuré pour effectuer une décontamination de l'air (30) jusqu'à une grandeur prédéfinie, dans lequel la grandeur prédéfinie est fixée de telle sorte que l'air peut également être utilisé dans l'agencement de dessiccation et de refroidissement par évaporation, dans lequel il est prévu d'éliminer des composés organiques volatils jusqu'à un certain degré d'au moins 70 % environ, et
- dans lequel l'air épuré est utilisé pour au moins un but du groupe constitué de :
- refroidir l'air d'entrée par récupération de chaleur, et
- dessécher un agent dessicatif de l'agencement de dessiccation et de refroidissement par évaporation, et **caractérisé en ce que**
les premières conduites d'alimentation en air sont prévues depuis l'agencement de dessiccation et de refroidissement par évaporation jusqu'au au moins un espace intérieur climatisé,
des conduites secondaires d'alimentation en air (36) sont prévues depuis le au moins un espace intérieur climatisé jusqu'à un agencement d'alimentation en air (38), et depuis l'agencement d'alimentation en air jusqu'au au moins un espace intérieur générant de l'air contaminé,
l'agencement de dessiccation et de refroidissement par évaporation est configuré pour conditionner de l'air extérieur frais en tant qu'air d'entrée en vue de l'utilisation dans l'espace intérieur climatisé,
l'agencement d'alimentation en air est configuré pour conditionner de l'air évacué (40) provenant du au moins un espace intérieur climatisé en vue de l'utilisation dans l'espace générant de l'air contaminé, et
les premières conduites d'alimentation en air sont configurées pour fournir l'air conditionné en tant qu'air d'alimentation au au moins un espace intérieur climatisé, et les conduites secondaires d'alimentation en air sont configurées pour diriger de l'air évacué (40) depuis le au moins un espace intérieur climatisé jusqu'à l'agencement d'alimentation en air, et pour fournir l'air conditionné en tant qu'air d'alimentation (44) au au moins un espace intérieur générant de l'air contaminé.

2. Système selon la revendication 1, dans lequel est prévu :
- au moins un espace intérieur climatisé, et/ou
- au moins un espace intérieur générant de l'air contaminé.

3. Système selon l'une des revendications précédentes, dans lequel l'agencement de dessiccation et de refroidissement par évaporation comporte un agent dessiccatif (118) fourni dans un générateur de sorption (120), et au moins une partie de la chaleur destinée à la récupération cyclique de l'agent dessiccatif est fournie par de l'énergie thermique à faible pouvoir calorifique (122) provenant d'une centrale mixte produisant de la chaleur et de l'électricité.

4. Système selon l'une des revendications précédentes, dans lequel l'agencement de dessiccation et de refroidissement par évaporation comporte :
- au moins une roue à dessiccation (124) avec un agent dessiccatif, laquelle roue à sec est agencée à l'intérieur de la première conduite d'alimentation en air avec une première portion (128) et à l'intérieur de la conduite secondaire de rejet avec une seconde portion (132), et
- au moins une roue de récupération de chaleur (134), qui est agencée à l'intérieur de la première conduite d'alimentation en air avec une première portion (136) et à l'intérieur de la conduite secondaire de rejet avec une seconde portion (138).

5. Système selon l'une des revendications précédentes, dans lequel au moins un dispositif de chauffage d'air (140) est prévu entre la roue de récupération de chaleur et la roue à dessiccation dans la conduite secondaire de rejet.

6. Système selon l'une des revendications précédentes, dans lequel l'épurateur d'air évacué est une unité intégrée de l'agencement de dessiccation et de refroidissement par évaporation.

7. Procédé (300) d'alimentation en air d'un site de production, comportant les étapes suivantes consistant à :
a) conditionner (310) de l'air frais extérieur dans un agencement de dessiccation et de refroidissement par évaporation en tant qu'air d'entrée en vue de l'utilisation dans l'espace intérieur générant de l'air contaminé, et fournir (312) l'air conditionné en tant qu'air d'alimentation à au moins un espace intérieur générant de l'air contaminé,
b) diriger (314) de l'air évacué contaminé depuis le au moins un espace intérieur générant de l'air contaminé jusqu'à un épurateur d'air évacué où de l'eau est pulvérisée pour i) humidifier et refroidir (318) l'air, et pour ii) effectuer (320) la décontamination de l'air jusqu'à une grandeur prédéfinie, dans lequel la grandeur prédéfinie est fixée de telle sorte que l'air peut également être utilisé dans l'agencement de dessiccation et de refroidissement par évaporation, dans lequel il est prévu d'éliminer des composés organiques volatils jusqu'à un certain degré d'au moins 70 % approximativement, et
c) fournir (322) l'air épuré à l'agencement de dessiccation et de refroidissement par évaporation, dans lequel l'air épuré est utilisé pour au moins un but du groupe consistant à :
- refroidir (326) l'air d'entrée par récupération de chaleur, et
- dessécher (328) un agent dessicatif de l'agencement de dessiccation et de refroidissement par évaporation,
**caractérisé en ce que** le site de production est constitué de différentes zones ayant différentes exigences de climatisation,
à l'étape a), l'air extérieur frais est conditionné (320) en vue de l'utilisation dans des espaces intérieurs climatisés, et l'air conditionné est délivré (332) en tant qu'air d'alimentation à au moins un espace intérieur climatisé,
entre l'étape a) et l'étape b), ces étapes sont prévues :
ab1) transfert (334) de l'air évacué depuis le au moins un espace intérieur climatisé jusqu'à un agencement d'alimentation en air d'espaces générant de l'air contaminé, et
ab2) conditionnement (336) de l'air évacué dans l'agencement d'alimentation en air en vue de l'utilisation dans des espaces générant de l'air contaminé, et fourniture (338) de l'air conditionné en tant qu'air d'alimentation au au moins un espace intérieur générant de l'air contaminé, et
à l'étape b), de l'air évacué contaminé provenant du au moins un espace intérieur générant de l'air contaminé est dirigé (340) vers l'épurateur d'air évacué.

8. Procédé selon la revendication 7, dans lequel l'air évacué des espaces intérieurs climatisés est utilisé en tant que source d'air pour des espaces générant de l'air contaminé, et
dans lequel l'air évacué des espaces intérieurs générant de l'air contaminé et/ou pour les espaces intérieurs climatisés est utilisé pour faire fonctionner l'agencement de dessiccation et de refroidissement par évaporation pour conditionner l'air d'alimentation de l'espace intérieur générant de l'air contaminé et/ou des espaces intérieurs climatisés.

9. Procédé selon la revendication 7 ou 8, dans lequel l'agencement de dessiccation et de refroidissement par évaporation comporte un agent dessiccatif fourni dans un générateur de sorption, et la récupération cyclique de l'agent dessiccatif est supportée par de l'énergie thermique à faible pouvoir calorifique provenant d'une centrale mixte produisant de la chaleur et de l'électricité.

10. Procédé selon l'une des revendications 7 à 9, dans lequel la contamination de l'aire est créée par :
i) des particules, dans lequel la quantité d'eau pulvérisée qui n'est pas dissoute dans l'air est au moins partiellement recueillie et fournie à un agencement de filtrage, et/ou
ii) des composés organiques volatils, dans lequel la quantité d'eau pulvérisée qui n'est pas dissoute dans l'air est au moins partiellement recueillie et fournie à un réacteur biologique, où des bactéries assurent la dégradation des composés organiques volatils en CO₂.

11. Procédé selon l'une des revendications 7 à 10,
dans lequel l'étape a) comporte :
- le séchage (346) de l'air d'entrée et le chauffage simultané de l'air d'entrée,
- le refroidissement (348) de l'air d'entrée chauffé par une roue de récupération de chaleur et l'humidification de l'air d'entrée,
- un chauffage supplémentaire facultatif (350),
dans lequel l'étape ab2) comporte :
- une humidification facultative (352),
- un chauffage supplémentaire facultatif (3 54), et
dans lequel l'étape b) comporte :
- l'humidification et l'épuration simultanées (358) en pulvérisant de l'eau à grande vitesse sur une surface où les gouttes d'eau sont atomisées en plus petites gouttelettes générant des charges négatives,
- l'absorption (360) de l'énergie thermique provenant de la roue de récupération de chaleur par l'air refroidi par l'humidification, et ainsi le réchauffement (362) de l'air,
- un chauffage facultatif (364) de l'air par un déshumidificateur à basse température,
- un chauffage facultatif (366) de l'air par un déshumidificateur à haute température en vue d'une désinfection, et
- le séchage (368) de l'agent dessiccatif avec de l'air chaud.

12. Procédé selon l'une des revendications 7 à 11,
dans lequel l'étape a) comporte :
- en premier lieu, l'humidification et le préchauffage (376) de l'air d'entrée avec une roue d'absorption,
- le chauffage (378) avec un dispositif de chauffage à haute température,
- un chauffage facultatif (380) avec un refroidisseur qui fonctionne également comme un dispositif de chauffage à basse température, en liaison avec l'épurateur, et
dans lequel l'étape ab2) comporte :
- l'augmentation (382) de la température de l'air par un chauffage par recirculation avec dispositif de refroidissement/chauffage, et
- une seconde humidification (3 84).

13. Procédé selon l'une des revendications 7 à 12,
dans lequel l'étape a) comporte :
- en premier lieu, l'humidification et le préchauffage (386) de l'air d'entrée avec une roue d'absorption,
- le chauffage (388) avec un dispositif de chauffage à haute température,
- un chauffage facultatif (390) avec un refroidisseur qui fonctionne également comme un dispositif de chauffage à basse température, en liaison avec l'épurateur,
- l'humidification et le refroidissement (392) avec un humidificateur d'entrée,
- le chauffage (394) avec un refroidisseur qui fonctionne également comme un dispositif de chauffage à basse température, et
dans lequel l'étape ab2) comporte :
- l'augmentation (396) de la température de l'air par un chauffage par recirculation avec dispositif de refroidissement/chauffage, et
- une seconde humidification (398).
